# EUROPEAN PATENT APPLICATION

(11) **EP 4 219 471 A1**
(43) Date of publication of application: **02.08.2023**
(21) Application number: 21886902.2
(22) Date of filing: 29.10.2021
(51) Int. Cl.: C07D 403/06, A61K 31/5377, A61P 3/04, A61P 3/10, A61P 29/00, A61P 15/10

(54) **AMORPHOUS MELANOCORTIN-4 RECEPTOR AGONIST**

(30) Priority: 29.10.2020 KR 20200142399
(71) Applicant: Lg Chem, Ltd., Seoul 07336 (KR)
(72) Inventor: HAM, Jin Ok, Daejeon 34122 (KR); LEE, Ho Yeon, Daejeon 34122 (KR); KIM, Ji Yoon, Daejeon 34122 (KR); KIM, Sung Won, Daejeon 34122 (KR); CHUN, Seul Ah, Daejeon 34122 (KR); LEE, Sang Dae, Daejeon 34122 (KR); PARK, Jong Won, Daejeon 34122 (KR)
(74) Representative: Hoffmann Eitle
(86) International application number: PCT/KR2021/015467
(87) International publication number: WO 2022/092908

(57) **Abstract**

The present invention relates to an amorphous compound represented by chemical formula 1, a preparation method therefor, and a pharmaceutical composition comprising same. The amorphous compound represented by chemical formula 1 according to the present invention may be characterized by an XRD pattern, a DSC profile, and/or a TGA profile.

## Description

### TECHNICAL FIELD

### Cross-reference to Related Applications

The present application claims the benefit of priority based on Korean Patent Application No. 10-2020-0142399, filed on 29 October 2020, the entire disclosure of which is incorporated as part of the specification.

### Technical Field

The present invention relates to a novel amorphous compound exhibiting an excellent agonistic activity for a melanocortin receptor, a method for preparing the same, and a pharmaceutical composition comprising the same.

### BACKGROUND ART

Leptin protein is a hormone secreted by adipocytes, and its secretion amount increases with an increase in body fat content. It regulates functions of various neuropeptides produced from hypothalamus, thereby regulating various in vivo functions, including appetite, body fat content, and energy metabolism (Schwartz, et al., Nature 404, 661-671 (2000)). The leptin protein signal transduction for controlling appetite and body weight is made through the regulation of many factors downstream, the most representative of which are melanocortin, agouti-related peptide (AgRP), and neuropeptide Y (NPY) hormones.

When the concentration of leptin in the blood increases as a result of excess calories in vivo, the secretion of proopiomelanocortin (POMC) protein hormone from the pituitary gland increases and the production of AgRP and NPY decreases. A small peptide hormone, alpha-melanocyte-stimulating hormone (MSH), is produced from POMC neurons. The hormone is an agonist for melanocortin-4 receptors (MC4R) of second-order neurons and ultimately induces appetite decrease. Meanwhile, when the concentration of leptin decreases as a result of calorie deficiency, the expression of AgRP, an MC4R antagonist, increases, and the expression of NPY also increases, which ultimately promotes appetite. That is, according to the change of leptin, the alpha-MSH hormone and the AgRP hormone act as agonists and antagonists for MC4R and thus are involved in appetite control.

The Alpha-MSH hormone binds to three MCR subtypes in addition to MC4R to induce various physiological reactions. Five MCR subtypes have been identified so far. Among them, MC1R is expressed mainly in skin cells and is involved in regulating melanin pigmentation (skin pigmentation). MC2R is expressed mainly in the adrenal gland and is known to be involved in the production of glucocorticoid hormones. Its ligand is only adrenocorticotropic hormone (ACTH) derived from POMC. MC3R and MC4R, which are expressed mainly in the central nervous system, are involved in regulating appetite, energy metabolism, and body fat storage efficiency, and MC5R expressed in various tissues is known to regulate exocrine function (Wikberg, et al., Pharm Res 42 (5) 393-420 (2000)). In particular, activation of the MC4R receptor induce appetite decrease and energy metabolism increase and thus has an effect of efficiently reducing body weight. Therefore, it has been proven to be a major action point in the development of anti-obesity drugs (Review: Wikberg, Eur. J. Pharmacol 375, 295-310 (1999)); Wikberg, et al., Pharm Res 42 (5) 393-420 (2000); Douglas et al., Eur J Pharm 450, 93-109 (2002); O'Rahilly et al., Nature Med 10, 351-352 (2004)).

The role of MC4R in the control of appetite and body weight was primarily demonstrated through an experiment in an animal model of abnormal expression of the agouti protein (agouti mouse). In the case of the Agouti mouse, it was found that due to genetic mutation, the agouti protein was expressed at a high concentration in the central nervous system and acted as an antagonist of MC4R in the hypothalamus to cause obesity (Yen, TT et al., FASEB J. 8, 479-488 (1994); Lu D., et al. Nature 371, 799-802 (1994)). Subsequent research results showed that the agouti-related peptides (AgRP) similar to the actual agouti protein were expressed in hypothalamic nerves, and these are also known to be antagonists for MC4R and be involved in controlling appetite (Shutter, et al., Genes Dev., 11, 593-602 (1997); Ollman, et al. Science 278, 135-138 (1997)).

Intracerebral administration of alpha-MSH, which is an in vivo MC4R agonist, to animals leads to the effect of reducing appetite. When treating the animals with SHU9119 (peptide) or HS014 (peptide), which are MC4R antagonists, it was observed that appetite increased again (Kask et al., Biochem. Biophys. Res. Comm. 245, 90-93 (1998)). In addition, in animal studies using Melanotan II (MTII, Ac-Nle-c[Asp-His-DPhe-Arg-Trp-Lys]-NH2) and the similar agonist thereof, HP228, after intracerebral, intraperitoneal, or subcutaneous administration, efficiencies of inhibiting appetite, reducing body weight, increasing energy metabolism, etc. were found. (Thiele T. E., et al. Am J Physiol 274 (1 Pt 2), R248-54 (1998); Lee M. D., et al. FASEB J 12, A552 (1998); Murphy B., et al. J Appl Physiol 89, 273-82 (2000)). On the contrary, administration of the representative SHU9119 to animals showed significant and sustained feed intake and weight gain, providing pharmacological evidence that MCR agonists could be anti-obesity agents. The effect of reducing appetite, which is clearly exhibited upon administration of MTII, was not observed in MC4R knock-out (KO) mice. This experimental result proves again that the appetite-reducing effect is achieved mainly through the activation of MC4R (Marsh, et al., Nat Genet 21, 119-122 (1999)).

Anorectic agents acting on the central nervous system were the main types of antiobestic drugs developed so far. Among them, most were drugs that modulate the action of neurotransmitters. Examples include noradrenalin agents (phentermine and mazindol), serotonergic agents, fluoxetine and sibutramine, and the like. However, the neurotransmitter modulators have a wide range of effects on various physiological actions in addition to appetite suppression, through numerous subtype receptors. Accordingly, the modulators lack selectivity for each subtype, and thus have a major disadvantage in that they are accompanied by various side effects when administered for a long period.

Meanwhile, melanocortin agonists are neuropeptides, not neurotransmitters. Given that in MC4R gene KO mice, all functions other than energy metabolism are normal, they have an advantage as an action point in that they can induce only weight loss through appetite suppression without affecting other physiological functions. In particular, the receptor is a G-protein coupled receptor (GPCR) that belongs to the most successful category of new drug action points developed so far. Thus, the action point greatly differs from existing action points in that it is relatively easy to secure selectivity for subtype receptors.

As an example of utilizing a melanocortin receptor as an action point, international publication nos. WO 2008/007930 and WO 2010/056022 disclose compounds as agonists of the melanocortin receptor.

In addition, the inventors of the present invention have conducted extensive studies and invented a novel compound of the following formula 1 having an excellent agonistic activity selective for a melanocortin receptor, in particular, melanocortin-4 receptor (MC4R), and a method for preparing the same (application no. KR 10-2019-0141649 (filed on 7 November 2019)): 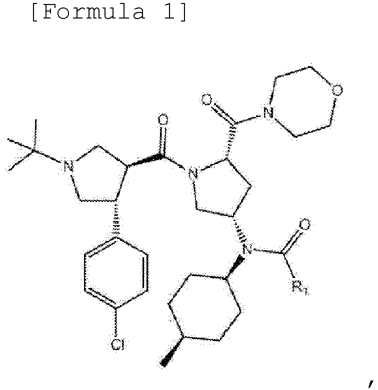 wherein R₁ is C₂-C₅ alkyl.

Meanwhile, the salt form or crystal structure of a pharmaceutically active ingredient often affects the chemical stability of the drug. Different salt forms of a compound can change the easiness of filtering the compound, product stability, storage stability, etc. Different crystallization conditions and storage conditions for the compound can lead to changes in the crystal structure of the compound and sometimes the accompanying production of other forms of the crystalline form. Accordingly, it is necessary to study salt forms and/or crystal structures having high purity and good chemical stability for a single compound.

### DISCLOSURE OF THE INVENTION

### TECHNICAL PROBLEM

An aspect of the present invention provides a novel amorphous compound having an excellent agonistic activity, which is selective for a melanocortin receptor, in particular, melanocortin-4 receptor (MC4R), and a salt thereof.

Another aspect of the present invention provides a method for preparing the amorphous compound or the salt thereof.

Another aspect of the present invention provides a pharmaceutical composition comprising the amorphous compound or the salt thereof.

### TECHNICAL SOLUTION

According to an aspect of the present invention, there is provided an amorphous compound of the following formula 1, or a salt thereof. 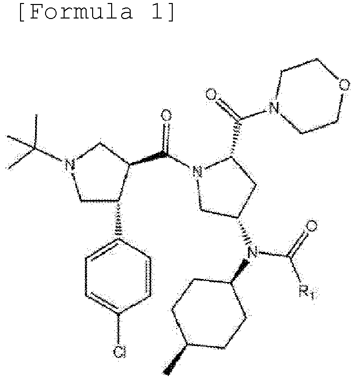 wherein R₁ is C₂-C₅ alkyl.

Since the compound according to the present invention can have an asymmetric carbon center and an asymmetric axis or an asymmetric plane, it can exist as cis or trans isomers, R or S isomers, racemates, diastereomer mixtures, and individual diastereomers, all of which are within the scope of the present invention.

In the present specification, unless otherwise specified for convenience, the compound of formula 1 is used to include all of the compound of formula 1, a pharmaceutically acceptable salt, and an isomer thereof.

In one embodiment according to the present invention, in formula 1, R₁ is C₂ to C₅ alkyl. In another embodiment according to the present invention, in formula 1, R₁ is a straight-chain or branched C₂ to C₅ alkyl, for example, ethyl, n-propyl, iso-propyl, n-butyl, iso-butyl, sec-butyl, or tert-butyl.

In another embodiment according to the present invention, in formula 1, R₁ is C₂ or C₃ alkyl. In another embodiment according to the present invention, in formula 1, R₁ is a straight-chain or branched C₂ or C₃ alkyl, for example, ethyl, n-propyl, or iso-propyl.

In one embodiment according to the present invention, the pharmaceutically acceptable salt includes, but are not limited to, acid-addition salts which are formed from inorganic acids, such as hydrochloric acid, sulfuric acid, nitric acid, phosphoric acid, hydrobromic acid, and hydroiodic acid; organic carbonic acids, such as tartaric acid, formic acid, citric acid, acetic acid, trichloroacetic acid, trifluoroacetic acid, gluconic acid, benzoic acid, lactic acid, fumaric acid, and maleic acid; or sulfonic acids, such as methanesulfonic acid, benzenesulfonic acid, p-toluenesulfonic acid, or naphthalene-sulfonic acid.

In another embodiment according to the present invention, the pharmaceutically acceptable salt of the amorphous compound of formula 1 may be a hydrochloride compound of the following formula 2: 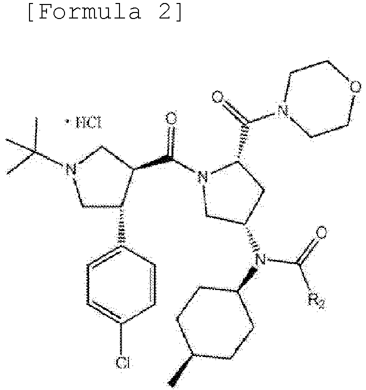 wherein R₂ is C₂-C₅ alkyl.

In another embodiment according to the present invention, the compound of formula 1 or the pharmaceutically acceptable salt thereof may be *N-*((3S,5S)-1-((3S,4R)-1-(*tert-*butyl)-4-(4-chlorophenyl)pyrrolidine-3-carbonyl)-5-(morpholine-4-carbonyl)pyrrolidin-3-yl)-*N*-((1*s*,4*R*)-4-methylcyclohexyl)isobutyramide hydrochloride of the following formula 3.

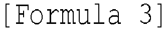

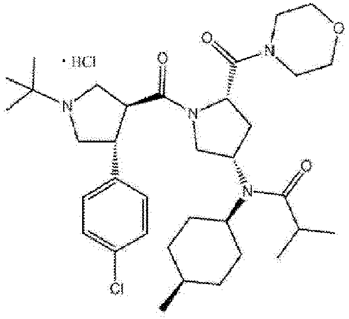

The X-ray diffraction (XRD) pattern of the amorphous compound or the pharmaceutically acceptable salt thereof, according to the present invention, may be confirmed to have no characteristic peak at a 20 angle of 4° to 40°.

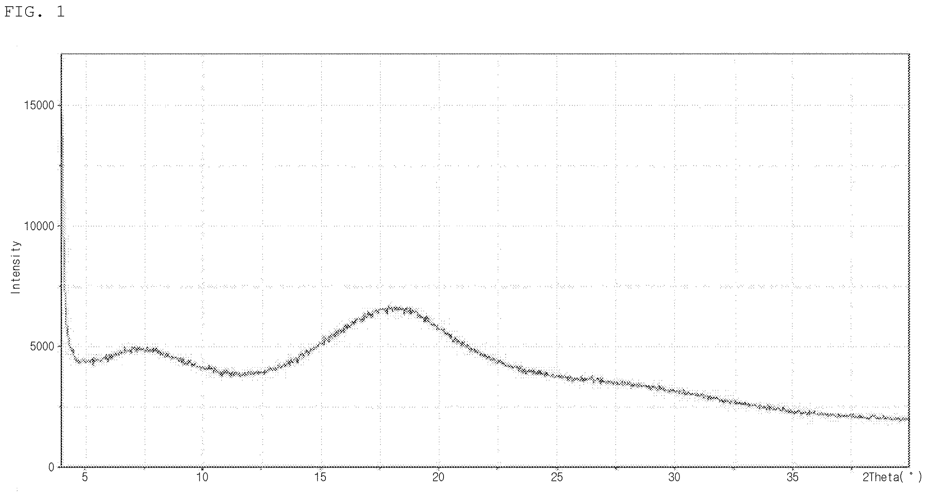

In one embodiment according to the present invention, the amorphous compound or the pharmaceutically acceptable salt thereof may have the XRD pattern shown in FIG. 1.

In the differential scanning calorimetry (DSC) profile, the amorphous compound or the pharmaceutically acceptable salt thereof, according to the present invention, may have no exothermic peak when heated to a temperature of 350°C or lower. In addition, in the DSC profile, the amorphous compound or the pharmaceutically acceptable salt thereof may have an endothermic peak at one or more of the temperature ranges of 20 to 130°C and 210 to 350°C. In addition, in the DSC profile, the amorphous compound or the pharmaceutically acceptable salt thereof may have a glass transition temperature (Tg) of 140°C to 165°C, for example, 150°C to 160°C, or 156°C.

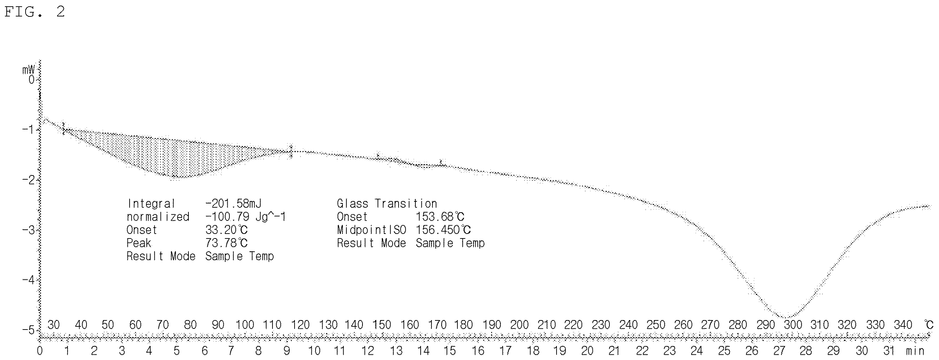

In one embodiment according to the present invention, the amorphous compound or the pharmaceutically acceptable salt thereof may have the DSC profile shown in FIG. 2.

The amorphous compound or the pharmaceutically acceptable salt thereof, according to the present invention, in the thermogravimetric analysis (TGA) profile, may have 10% or less, for example, 1% to 10%, 5% to 9%, or 7% of weight loss when heated to a temperature of 180°C or less.

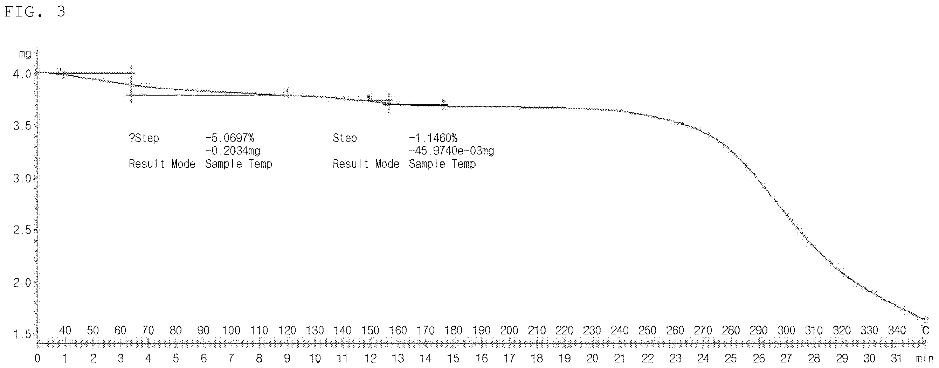

In one embodiment according to the present invention, the amorphous compound or the pharmaceutically acceptable salt thereof may have the TGA profile shown in FIG. 3.

In the present specification,

X-ray diffraction (XRD) analysis shows the results performed using PANalytical X' Pert Pro MPD system (Malvern Panalytical Ltd.).

Differential scanning calorimetry (DSC) analysis shows the results performed using DSC1 (Mettler-Toledo AG).

Thermogravimetric analysis (TGA) shows the results performed using TGA/DSC 1 (Mettler-Toledo AG).

Stability analysis shows the results performed using HPLC (Agilent Technologies, Inc.).

The amorphous compound of formula 1 or the pharmaceutically acceptable salt thereof may have higher purity than a crude compound or pharmaceutically acceptable salts thereof and be physically and chemically more stable.

In addition, the compound of formula 2 is a hydrochloride form of the compound of formula 1, and the amorphous hydrochloride compound of formula 2 may have higher purity than the compound of formula 1 and be physically and chemically more stable. The agonistic ability for the melanocortin-4 receptor and preventive or therapeutic effects on diseases, such as obesity, diabetes, inflammation, erectile dysfunction, or the like can be more excellent than those of known melanocortin-4 receptor agonists. However, the effects of the present invention are not limited thereto.

In another aspect, the present invention provides a method for preparing the amorphous compound of formula 1 or the pharmaceutically acceptable salt thereof, comprising the steps of: preparing a mixed solution by dissolving the crude compound represented by formula 1 in an organic solvent; cooling the mixed solution; and adding an acid to the cooled mixed solution.

First, the crude compound represented by formula 1 is dissolved in an organic solvent.

The crude compound of formula 1, for preparing the amorphous compound of formula 1 or the pharmaceutically acceptable salt thereof, may be a compound of formula 1, a salt, or an isomer thereof.

The compound of formula 1 may be obtained by the preparation method described in the specification of application no. KR 10-2019-0141649 (filed on 7 November 2019) .

The solvent for dissolving the crude compound of formula 1 may be used without particular limitation as long as it is a solvent capable of dissolving the crude compound of formula 1. In one embodiment, the solvent may be an organic solvent. Specifically, the solvent may include an ether-based organic solvent.

Examples of the ether-based organic solvent include, but are not limited to, dialkyl ether-based solvents, such as diethyl ether, dipropyl ether, dibutyl ether, diisoamyl ether, ethyl methyl ether, methyl propyl ether, methyl butyl ether, and ethyl propyl ether; cyclic ether-based solvents, such as tetrahydrofuran and tetrahydropyran; and aromatic ring-containing ether-based solvents, such as, diphenyl ether and anisole; and so on. As for the organic solvent, one solvent may be used alone, or two or more solvents may be mixed and used.

In one embodiment according to the present invention, the organic solvent may include methyl tert-butyl ether (MTBE).

For 1 g of the crude compound of formula 1, 10 to 30 mL, or 15 mL to 25 mL of the organic solvent may be used.

Dissolution of the crude compound of formula 1 in the solvent may be carried out without or with stirring at a temperature of 15 to 30°C, specifically 23 to 28°C.

In one embodiment according to the present invention, a mixed solution in which the crude compound of formula 1 has been dissolved at 25°C may be obtained by using 19 mL of MTBE with respect to 1 g of the crude compound of formula 1.

Next, the mixed solution in which the crude compound of formula 1 has been dissolved is cooled.

The cooling may be performed so that the temperature of the mixed solution becomes 0°C or lower. Specifically, the cooling may be performed so that the temperature of the mixed solution becomes -10 to 0°C, specifically, -5 to 0°C.

The preparation method of the present invention may further comprise a step for adding a non-polar organic solvent to the mixed solution before, after, or simultaneously with cooling the mixed solution. The yield or production stability of the obtained amorphous compound may be improved by adding the non-polar organic solvent to increase the production rate of crystallized particles, but the present invention is not limited thereto.

The non-polar organic solvent may be used without particular limitation as long as it is an organic solvent having non-polar properties, but, for example, hexane, heptane, cyclohexane, carbon tetrachloride, benzene, chloroform, and the like, may be used.

In one embodiment, according to the present invention, heptane may be added to the mixed solution, and then, the mixed solution may be cooled to -5 to 0°C.

The step for adding the non-polar organic solvent may be any step in the preparation method for the amorphous compound of the present invention.

Next, an acid is added dropwise to the cooled mixed solution.

The acid includes hydrochloric acid. The adding the acid dropwise may be carried out in the presence of ethyl acetate.

In one embodiment according the present invention, the adding the acid dropwise may be adding a hydrochloric acid-ethyl acetate solution to the mixed solution.

The concentration of hydrochloric acid in the hydrochloric acid-ethyl acetate solution may be 0.5 M to 6 M, specifically 1 M to 5 M, or 3 M to 5 M, for example, 4 M.

A precipitate formed by adding the acid dropwise may be filtered and washed to obtain the amorphous compound of formula 1, specifically, the amorphous compound of formula 2, or a pharmaceutically acceptable salt of the amorphous compounds.

The amorphous compound of formula 1 as obtained above may have higher purity than a crude compound of formula 1 and may be physically and chemically more stable. However, the effects of the present invention are not limited thereto.

In another aspect, the present invention provides a pharmaceutical composition comprising: (i) the amorphous compound of formula 1, or the salt of the amorphous compound of formula 1; and (ii) a pharmaceutically acceptable carrier.

The amorphous compound of formula 1, or the salt of the amorphous compound of formula 1, according to the present invention, exhibits excellent agonistic actions on melanocortin receptors, in particular, melanocortin-4 receptors (MC4R). Thus, the present invention can provide a pharmaceutical composition for agonizing melanocortin receptors, the composition containing the above-described amorphous compound or the salt thereof as an active ingredient. Specifically, the pharmaceutical composition may be a composition for agonizing the function of the melanocortin-4 receptor.

In addition, since the pharmaceutical composition can exhibit excellent effects of preventing or treating obesity, diabetes, inflammation, and erectile dysfunction, it may be a composition for preventing or treating obesity, diabetes, inflammation, or erectile dysfunction. However, the use of the present invention is not limited the diseases.

As used herein, "carrier" refers to a compound that facilitates the introduction of compounds into a cell or tissue.

When the amorphous compound of the present invention is administered for clinical purposes, the total daily dose to be administered to a host in a single dose or in divided doses may be preferably in the range of 0.01 to 10 mg/kg body weight. However, the specific dose level for an individual patient may vary depending on the specific compound to be used, the patient's weight, sex, health status, diet, administration time of the drug, administration method, excretion rate, drug combination, the severity of the disease, or the like.

The amorphous compound of the present invention may be administered by any route as desired. For example, the amorphous compound of the present invention may be administered by injection or orally.

The pharmaceutical composition of the present invention may be in various oral dosage forms, such as tablets, pills, powders, capsules, granules, syrups, or emulsions, or parenteral dosage forms, such as injection preparations for intramuscular, intravenous, or subcutaneous administration.

Preparations for injection may be prepared according to known techniques using suitable dispersing agents, wetting agents, suspending agents, or excipients.

Excipients that can be used in the pharmaceutical preparation of the present invention include, but are not limited to, sweeteners, binders, solubilizers, solubilizing agents, wetting agents, emulsifiers, isotonic agents, adsorbents, disintegrants, antioxidants, preservatives, lubricants, fillers, fragrances, etc. For example, as excipients, lactose, dextrose, sucrose, mannitol, sorbitol, cellulose, glycine, silica, magnesium aluminum silicate, starch, gelatin, gum tragacanth, arginic acid, sodium alginate, methylcellulose, sodium carboxymethyl cellulose, water, ethanol, polyethylene glycol, polyvinyl pyrrolidone, sodium chloride, calcium chloride, orange essence, strawberry essence, vanilla flavor, etc. may be used.

When the pharmaceutical composition of the present invention is in an oral dosage form, examples of the carrier to be used may include, but are not limited to, cellulose, calcium silicate, corn starch, lactose, sucrose, dextrose, calcium phosphate, stearic acid, magnesium stearate, calcium stearate, gelatin, talc, etc.

When the pharmaceutical composition of the present invention is in an injectable preparation form, examples of the carrier may include, but are not limited to, water, saline, aqueous glucose solution, an aqueous sugar-like solution, alcohols, glycol, ethers, oils, fatty acids, fatty acid esters, glycerides, etc.

In another aspect, there is provided an amorphous compound of formula 1, or a salt compound of the amorphous compound as described above for use in agonizing the functions of melanocortin receptors, in particular, melanocortin-4 receptors (MC4R).

In one embodiment, there is provided an amorphous compound of formula 1, or a salt compound of the amorphous compound as described above for use in treating or preventing obesity, diabetes, inflammation, or erectile dysfunction.

In another aspect, there is provided a method for agonizing the function of melanocortin receptors, in particular, melanocortin-4 receptors (MC4R), the method comprising a step for administering to a subject the amorphous compound of formula 1, or the salt compound of the amorphous compound as described above.

In another aspect, there is provided a method for treating obesity, diabetes, inflammation, or erectile dysfunction, the method comprising a step for administering to a subject the amorphous compound of formula 1, or the salt compound of the amorphous compound as described above.

### ADVANTAGEOUS EFFECTS

The amorphous compound of formula 1 or the salt thereof, according to the present invention, exhibits excellent agonistic action on melanocortin receptors, in particular, melanocortin-4 receptors (MC4R), and thus can be usefully used for preventing or treating obesity, diabetes, inflammation, and erectile dysfunction.

The amorphous compound of formula 1 or the salt thereof, according to the present invention, exhibits an on-target effect on melanocortin-4 receptors, thereby exhibiting weight loss and diet reduction effects, without affecting anxiety and depression. In addition, it can be administered without any safety issues, such as side effects of human ether-a-go-go related gene (hERG) inhibition or mutagenesis.

In addition, the amorphous compound of formula 1 or the salt thereof, according to the present invention, has purity, yield, physical and chemical stability, which are more excellent than the crude compound of formula 1.

Specifically, the amorphous compound of formula 1 or the salt thereof may have superior solubility, storage stability, and production stability to the crude compound of formula 1.

### BRIEF DESCRIPTION OF THE DRAWINGS

FIG. 1 is the graph of the XRD result of Example 1.
FIG. 2 is the graph of the DSC result of Example 1.
FIG. 3 is the graph of the TGA result of Example 1.

### MODE FOR CARRYING OUT THE INVENTION

Hereinafter, the present invention will be described in more detail through Preparation Examples and Examples. However, these Examples are merely illustrative of the present invention, and the scope of the present invention is not limited thereto.

### Preparation Example 1: Preparation of methyl (2S,4S)-4-(N-((1s,4R)-4-methylcyclohexyl)isobutyramido)pyrrolidine-2-carboxylate hydrochloride

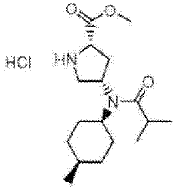

The title compound was obtained through the following steps A, B, C, D, and E.

### Step A: Preparation of 1-(tert-butyl) 2-methyl (2S,4S)-4-azidopyrrolidine-1,2-dicarboxylate

1-(tert-butyl) 2-methyl (2S,4R)-4-((methylsulfonyl)oxy)pyrrolidine-1,2-dicarboxylate (48.5 g, 150 mmol) was dissolved in N,N'-dimethylformamide (250 ml) under nitrogen, and sodium azide (19.5 g, 300 ml) was added. After stirring at 80°C for 16 hours, the reaction solvent was concentrated under reduced pressure, water was added, and extraction was performed twice with ethyl acetate. The organic layer was washed with an aqueous sodium chloride solution and water, dried over anhydrous magnesium sulfate, and filtered. The filtrate was concentrated under reduced pressure to obtain crude 1-(tert-butyl) 2-methyl (2S,4S)-4-azidopyrrolidine-1,2-dicarboxylate (39.59 g, 98%), which was used in the next step without purification.

MS [M+H] = 271 (M+1)

¹H NMR (400 MHz, CD₃OD) δ 4.43-4.37 (m, 1H), 4.35-4.27 (br, 1H), 3.77 (s, 1.8H), 3.76 (s, 1.2H), 3.73-3.66 (m, 1H), 3.44-3.38 (m, 1H), 2.63-2.49 (m, 1H), 2.19-2.11 (m, 1H), 1.50 (s, 4.5H), 1.44 (s, 4.5H)

### Step B: Preparation of 1-(tert-butyl) 2-methyl (2S,4S)-4-aminopyrrolidine-1,2-dicarboxylate

1-(tert-butyl) 2-methyl (2S,4S)-4-azidopyrrolidine-1,2-dicarboxylate (24.59 g, 91.0 mmol) obtained in step A above was dissolved in tetrahydrofuran (180 ml), and 1 M trimethylphosphine tetrahydro solution (109.2 ml, 109.2 mmol) was slowly added at 0°C. After stirring at the same temperature for 1 hour, the mixture was stirred at room temperature for 3 hours. After the reaction solvent was concentrated under reduced pressure, dichloromethane (100 ml) and water (150 ml) were added, and the mixture was stirred for about 30 minutes. The layers were separated and were extracted once more with dichloromethane, and the organic layer was dried over anhydrous magnesium sulfate and was filtered. The filtrate was concentrated under reduced pressure to obtain crude 1-(tert-butyl) 2-methyl (2S,4S)-4-aminopyrrolidine-1,2-dicarboxylate (20.62 g, 93%), which was used in the next step without purification.

MS [M+H] = 245 (M+1)

¹H NMR (400 MHz, CD₃OD) δ 4.27 (m, 1H), 3.77 (s, 1.8H), 3.76 (s,1.2H), 3.75-3.67 (m, 1H), 3.50-3.42 (m, 1H), 3.22-3.17 (m, 1H), 2.58-2.47 (m,1H), 1.82-1.71 (m, 1H), 1.48 (s, 4.5H), 1.42 (s, 4.5H)

### Step C: Preparation of 1-(tert-butyl) 2-methyl (2S,4S)-4-(((1s,4R)-4-methylcyclohexyl)amino)pyrrolidine-1,2-dicarboxylate

1-(tert-butyl) 2-methyl (2S,4S)-4-aminopyrrolidine-1,2-dicarboxylate (20.62 g, 84.4 mmol) obtained in step B above was dissolved in dichloroethane (150 ml), and 4-methylcyclohexanone (9.5 ml, 101.3 mmol) was added. Sodium triacetoxyborohydride (26.8 g, 126.6 mmol) was added at 0°C, and the mixture was stirred at room temperature for 16 hours. The reaction solvent was concentrated under reduced pressure, water was added, and extraction was performed twice with ethyl acetate. The organic layer was washed with an aqueous sodium chloride solution, dried over anhydrous magnesium sulfate, and filtered. The filtrate was concentrated under reduced pressure and purified by column chromatography to obtain 1-(tert-butyl) 2-methyl (2S,4S)-4-(((1s,4R)-4-methylcyclohexyl)amino)pyrrolidine-1,2-dicarboxylate (22.9 g, 80%) .

MS [M+H] = 341 (M+1)

¹H NMR (400 MHz, CD₃OD) δ 4.26 (m, 1H), 3.76 (s, 1.8H), 3.75 (s, 1.2H), 3.78-3.71 (m, 1H), 3.49-3.40 (m, 1H), 3.22-3.16 (m, 1H), 2.69-2.60 (br, 1H), 2.58-2.46 (m, 1H), 1.87-1.77 (m, 1H), 1.73-1.63 (m, 1H), 1.62-1.35 (m, 8H), 1.48 (s, 4.5H), 1.42 (s, 4.5H), 0.96 (d, 3H)

### Step D: Preparation of 1-(tert-butyl) 2-methyl (2S,4S)-4-(N-((1s,4R)-4-methylcyclohexyl)isobutyramido)pyrrolidine-1,2-dicarboxylate

1-(tert-butyl) 2-methyl (2S,4S)-4-(((1s,4R)-4-methylcyclohexyl)amino)pyrrolidine-1,2-dicarboxylate obtained in step C above (37.29 g, 109.5 mmol) was dissolved in dichloromethane (500 ml), triethyl amine (61.1 ml, 438.1 mmol) was added, and then isobutyryl chloride (11.7 ml, 219 mmol) was slowly added at 0°C. After stirring at room temperature for 16 hours, the reaction solvent was concentrated under reduced pressure, an aqueous sodium hydrogen carbonate solution was added, and extraction was performed twice with ethyl acetate. The organic layer was washed with an aqueous sodium chloride solution and water, dried over anhydrous magnesium sulfate, and filtered. The filtrate was concentrated under reduced pressure, and purified by column chromatography to obtain 1-(tert-butyl) 2-methyl (2S,4S)-4-(N-((1s,4R)-4-methylcyclohexyl)isobutyramido)pyrrolidine-1,2-dicarboxylate (38.79 g, 86%).

MS [M+H] = 411 (M+1)

¹H NMR (400 MHz, CD3OD) δ 4.27 (m, 1H), 3.76 (s, 1.8H), 3.75 (s, 1.2H), 3.78-3.72 (m, 1H), 3.50-3.41 (m, 1H), 3.33-3.14 (m, 1H), 2.69-2.60 (m, 2H), 2.57-2.43 (m, 1H), 1.87-1.79 (m, 1H), 1.70-1.61 (m, 1H), 1.60-1.32 (m, 8H), 1.47 (s, 4.5H), 1.41 (s, 4.5H), 1.10 (dd, 6H), 0.99 (d, 3H)

### Step E: Preparation of methyl (2S,4S)-4-(N-((1s,4R)-4-methylcyclohexyl)isobutyramido)pyrrolidine-2-carboxylate hydrochloride

1-(tert-butyl) 2-methyl (2S,4S)-4-(N-((1s,4R)-4-methylcyclohexyl)isobutyramido)pyrrolidine-1,2-dicarboxylate (34.0 g, 82.8 mmol) obtained in step D above was dissolved in dichloromethane (200 ml), and a solution of 4 N hydrochloric acid in 1,4-dioxane solution (82.8 ml, 331.3 mmol) was added at 0°C. After stirring at room temperature for 6 hours, the reaction solvent was concentrated under reduced pressure to obtain crude (28.7 g, 99%), which was used in the next step without purification.

MS[M+H] = 311 (M+1)

### Preparation Example 2: Preparation of (3S,4R)-1-(tert-butyl)-4-(4-chlorophenyl)pyrrolidine-3-carboxylic acid

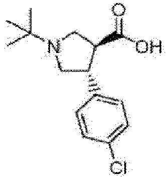

The title compound was obtained according to the method described in international patent publication no. WO 2004/092126.

MS[ M+H] = 282 (M+1)

¹H NMR (400 MHz, CD₃OD) δ 7.43-7.33 (m, 4H), 3.90-3.69 (m, 3H), 3.59 (dd, J = 11.2, 10.0 Hz, 1H), 3.29 (dd, J = 11.2, 11.2 Hz, 1H), 3.18-3.09 (m, 1H), 1.44 (s, 9H)

### Preparation Example 3: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide

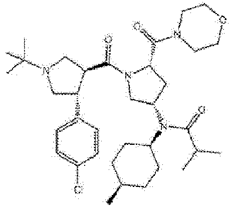

### Preparation Example 3 (MC70)

The title compound was obtained through the following steps A, B, and C.

### Step A: Preparation of methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate

Methyl (2S,4S)-4-(N-((1s,4R)-4-methylcyclohexyl)isobutyramido)pyrrolidine-2-carboxylate hydrochloride (28.7 g, 82.73 mmol) obtained in Preparation Example 1, (3S,4R)-1-(tert-butyl)-4-(4-chlorophenyl)pyrrolidine-3-carboxylic acid (24.5 g, 86.87 mmol) obtained in Preparation Example 2, 1-(3-dimethylaminopropyl)-3-ethylcarbodiimide hydrochloride (22.2 g, 115.83 mmol), and 1-hydroxybenzotriazole hydrate (15.7 g, 115.83 mmol) were dissolved in N,N'-dimethylformamide (400 ml), and N,N'-diisopropylethylamine (72.0 ml, 413.66 mmol) was added slowly. After stirring at room temperature for 16 hours, the reaction solvent was concentrated under reduced pressure, 0.5 N sodium hydroxide aqueous solution was added, and extraction was performed twice with ethyl acetate. The organic layer was washed twice with sodium chloride aqueous solution and water, dried over anhydrous magnesium sulfate, and filtered. The filtrate was concentrated under reduced pressure and purified by column chromatography to obtain methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate (41.19 g, 87%).

MS [M+H] = 575 (M+1)

### Step B: Preparation of (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylic acid

Methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate (39.4 g, 68.62 mmol) obtained in step A above was dissolved in methanol (450 ml), and then, 6 N sodium hydroxide aqueous solution (57.2 ml, 343.09 mmol) was added. After stirring at room temperature for 16 hours, and adjusting the pH to about 5 with 6 N aqueous hydrochloric acid solution, the reaction solution was concentrated under reduced pressure. After dissolving the concentrate in dichloromethane, the insoluble solid was filtered through a paper filter. The filtrate was concentrated under reduced pressure to obtain the crude title compound (38.4 g, 99%), which was used in the next step without purification.

MS [M+H] = 561 (M+1)

### Step C: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide

(2S,4S)-1-((3S,4R)-1-(tert-butyl)-4-(4-chlorophenyl)pyrrolidine-3-carbonyl)-4-(N -((1s,4R)-4-methylcyclohexyl)isobutyramido)pyrrolidine-2-carboxylic acid (38.4 g, 68.60 mmol) obtained in step B above, 1-(3-dimethylaminopropyl)-3-ethylcarbodiimide hydrochloride (18.4 g, 96.04 mmol), and 1-hydroxybenzotriazole hydrate (13.0 g, 96.04 mmol) were dissolved in N,N'-dimethylformamide (200ml), and then morpholine (5.9 ml, 68.80 mmol) and N,N'-diisopropylethylamine (59.7 ml, 343.02 mmol) were sequentially and slowly added. After stirring at room temperature for 16 hours, the reaction solution was concentrated under reduced pressure, 0.5 N sodium hydroxide aqueous solution was added, and extraction was performed twice with ethyl acetate. The organic layer was washed twice with sodium chloride aqueous solution and water, dried over anhydrous magnesium sulfate, and filtered. The filtrate was concentrated under reduced pressure and purified by column chromatography to obtain N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide (37.05 g, 86%, Preparation Example 3).

MS [M+H] = 630 (M+1)

### Example 1

### Preparation of amorphous compound of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide hydrochloride

Based on 1 g of the compound (MC70) finally prepared in Preparation Example 3 above, 19 mL of MTBE was used to dissolve the compound (MC70) at 25°C. After dissolution was completed, 1 mL of heptane was added, and then the mixture was cooled to -5 to 0°C. After the set temperature was reached, 1 equivalent of 4 M HCl/EtOAc was added dropwise, the mixture was stirred for about 90 minutes, and filtered to obtain amorphous N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide hydrochloride (Example 1). (yield: about 90%)

### Amorphous Characteristics

### 1) X-ray Diffraction (XRD)

The powder XRD diffraction pattern was obtained using PANalytical X'Pert Pro MPD system equipped with a monochromatized radiation source and Ni filter as a solid-state detector by the following method.

About 20 to 30 mg of the sample was compressed in a glass sample holder so that the sample had a flat surface, the generator of the apparatus was set to 45 kV (acceleration voltage) and 40 mA (filament emission), and then, the measurement was carried out in a reflection mode (not-spin). Bragg angles (2θ) in a range of 4 to 40° were measured with the conditions of step size of 0.026° and time per step of 51 seconds.

The XRD result is shown in FIG. 1. As shown in FIG. 1, amorphous Example 1 did not have no diffraction peaks and exhibited broad noises, typical of amorphous samples.

### 2) Differential Scanning Calorimetry (DSC)

The DSC was measured using Mettler Toledo DSC1 system. 2-5 mg of the sample is weighed and put into a 40 µL Al crucible (flat-bottomed aluminum pan with one pin-hole lid), and one pinhole is made. Then, DSC measurement is performed while the sample is heated from 25°C to 350°C at a rate of 10°C/min. During the measurement, nitrogen gas is supplied to the inside of the instrument at a rate of 70 mL/min to prevent the inflow of oxygen and other gases. Data collection and evaluation were performed using the software STARe.

The DSC result is shown in FIG. 2. An endothermic peak was observed at about 32.2°C (onset). A glass transition temperature was observed at about 156.5°C (Midpoint ISO) . After about 220°C, an endothermic peak due to decomposition appeared. The temperature values have an error of ±5°C.

### 3) Thermogravimetric Analysis (TGA)

The TGA was measured using Mettler Toledo TGA/DSC 1 module. About 4-8 mg of the sample is weighed and put into a 100 µL Al crucible (flat-bottomed aluminum crucibles). Then, TGA measurement is performed while the sample is heated from 30°C to 350°C at a rate of 10°C/min. During the measurement, nitrogen gas is supplied to the inside of the instrument at a rate of 80 mL/min to prevent the inflow of oxygen and other gases. Data collection and evaluation were performed using the software STARe.

The TGA result is shown in FIG. 3. As shown in FIG. 3, a weight loss of about 5.6% was observed at a temperature of less than 100°C. After that, a weight loss of about 1.1% was observed at about 150°C-180°C. After about 220°C, a weight loss due to decomposition occurred. The temperature values have an error of ±5°C.

### Experimental Example 1. Stability assessment

About 10-30 mg of the sample was stored for 4 weeks in an open state under the accelerated condition (40°C, 75% RH) or in a sealed state under the harsh condition in an oven at 80°C. To compare the samples with the samples stored at room temperature, HPLC analysis was performed by the method shown in Table 1 below.

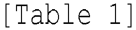

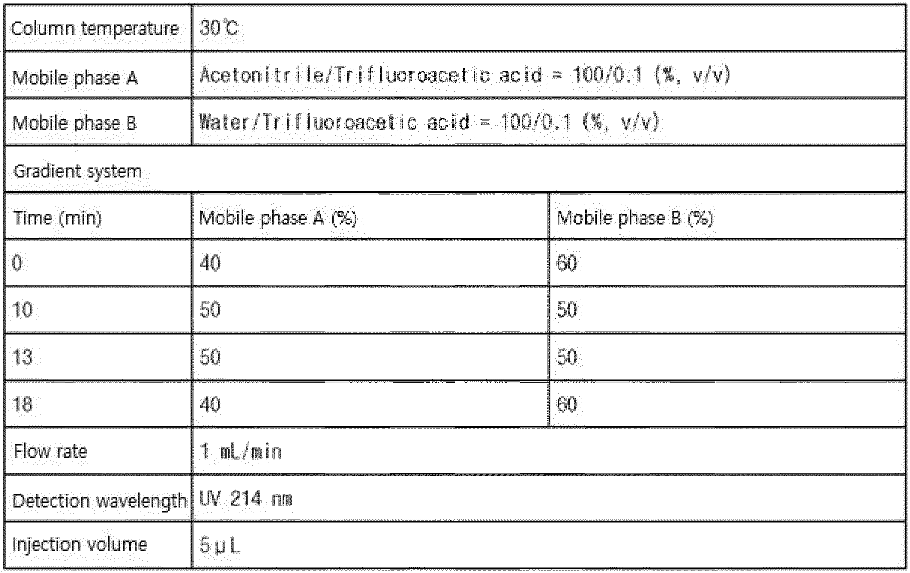

The stability result for Example 1 as evaluated according to the above method is shown in Table 2 below.

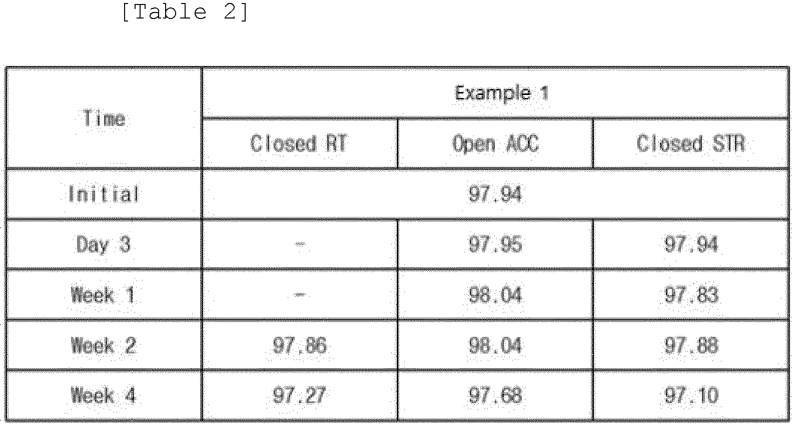

## Claims

1. An amorphous compound of the following formula 1, or a pharmaceutically acceptable salt thereof: 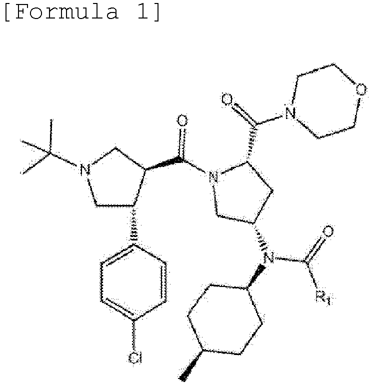 wherein R₁ is C₂-C₅ alkyl.

2. The compound or the pharmaceutically acceptable salt thereof of claim 1, having the X-ray diffraction pattern shown in FIG. 1.

3. The compound or the pharmaceutically acceptable salt thereof of claim 1, having the DSC profile shown in FIG. 2.

4. The compound or the pharmaceutically acceptable salt thereof of claim 1, having the TGA profile shown in FIG. 3.

5. The compound or the pharmaceutically acceptable salt thereof of claim 1, wherein the pharmaceutically acceptable salt of the compound of formula 1 is selected from the group consisting of: hydrochloric acid, sulfuric acid, nitric acid, phosphoric acid, hydrobromic acid, and hydroiodic acid, of the compound.

6. The compound or the pharmaceutically acceptable salt thereof of claim 1, which is *N-((35,55)-1-((35,4R)-1-(tert-*butyl)-4-(4-chlorophenyl)pyrrolidine-3-carbonyl)-5-(morpholine-4-carbonyl)pyrrolidin-3-yl)-*N*-((1s,4R)-4-methylcyclohexyl)isobutyramide hydrochloride.

7. A method for preparing the amorphous compound or the pharmaceutically acceptable salt thereof described in any one of claims 1-6, the method comprising the steps of:
preparing a mixed solution by dissolving the compound represented by the following formula 1 in an organic solvent;
cooling the mixed solution; and
adding an acid to the cooled mixed solution dropwise: 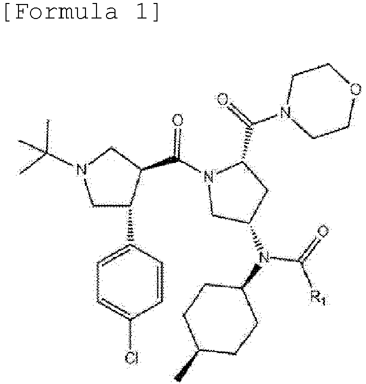 wherein R₁ is C₂-C₅ alkyl.

8. The preparation method of claim 7, wherein the organic solvent includes an ether-based organic solvent.

9. The preparation method of claim 7, further comprising a step for adding a non-polar organic solvent to the mixed solution before, after, or simultaneously with cooling the mixed solution.

10. A pharmaceutical composition comprising the compound according to any one of claims 1-6 and a pharmaceutically acceptable carrier.

11. A pharmaceutical composition for agonizing the function of a melanocortin-4 receptor, the composition comprising the compound according to any one of claims 1-6 and a pharmaceutically acceptable carrier.

12. The pharmaceutical composition of claim 11, which is for preventing or treating obesity, diabetes, inflammation, or erectile dysfunction.
